# EUROPEAN PATENT SPECIFICATION

(11) **EP 1 725 575 B1**
(45) Date of publication and mention of the grant of the patent: **28.11.2012**
(21) Application number: 05713397.7
(22) Date of filing: 14.02.2005
(51) Int. Cl.: C07K 1/00, C07K 2/00, A61K 39/02, A61K 39/00, A61K 39/085, A61K 39/09

(54) **POLYPEPTIDES FOR INDUCING A PROTECTIVE IMMUNE RESPONSE AGAINST STAPHYLOCOCCUS AUREUS**
POLYPEPTIDE ZUR INDUKTION EINER PROTEKTIVEN IMMUNANTWORT GEGEN STAPHYLOCOCCUS AUREUS
POLYPEPTIDES D'INDUCTION D'UNE REPONSE IMMUNE PROTECTRICE CONTRE STAPHYLOCOCCUS AUREUS

(30) Priority: 18.02.2004 US 545447 P
(43) Date of publication of application: 29.11.2006
(73) Proprietor: Merck Sharp & Dohme Corp., Rahway, NJ 07065-0907 (US)
(72) Inventor: ANDERSON, Annaliesa, S., Rahway, New Jersey 07065-0907 (US); MONTGOMERY, Donna, L., Rahway, New Jersey 07065-0907 (US)
(74) Representative: Horgan, James Michael Frederic
(86) International application number: PCT/US2005/004431
(87) International publication number: WO 2005/079315

(56) References cited:
- WO-A-01/98499
- WO-A-02/059148
- WO-A-02/094868
- WO-A-02/102829
- WO-A2-03/011899
- KURODA M ET AL: "Whole genome sequencing of meticillin-resistant Staphylococcus aureus." LANCET 21 APR 2001, vol. 357, no. 9264, 21 April 2001 (2001-04-21), pages 1225-1240, XP002511975 ISSN: 0140-6736
- MAZMANIAN SARKIS K ET AL: "Sortase-catalysed anchoring of surface proteins to the cell wall of Staphylococcus aureus" MOLECULAR MICROBIOLOGY, WILEY-BLACKWELL PUBLISHING LTD, GB, vol. 40, no. 5, 1 June 2001 (2001-06-01), pages 1049-1057, XP002361153 ISSN: 0950-382X
- TAYLOR J M ET AL: "Transferrin binding in Staphylococcus aureus: involvement of a cell wall-anchored protein" MOLECULAR MICROBIOLOGY, WILEY-BLACKWELL PUBLISHING LTD, GB, vol. 43, no. 6, 1 January 2002 (2002-01-01), pages 1603-1614, XP003004471 ISSN: 0950-382X
- MAZMANIAN S K ET AL: "AN IRON-REGULATD SORTASE ANCHORS A CLASS OF SURFACE PROTEIN DURING STAPHYLOCOCCUS AUREUS PATHOGENESIS" PROCEEDINGS OF THE NATIONAL ACADEMY OF SCIENCES OF USA, NATIONAL ACADEMY OF SCIENCE, WASHINGTON, DC.; US, vol. 99, no. 4, 19 February 2002 (2002-02-19), pages 2293-2298, XP001156903 ISSN: 0027-8424

## Description

### CROSS-REFERENCE TO RELATED APPLICATIONS

The present application claims the benefit of U.S. Provisional Application No. 60/545,447, filed February 18, 2004, which is hereby incorporated by reference herein.

### BACKGROUND OF THE INVENTION

The references cited throughout the present application are not admitted to be prior art to the claimed invention.

*Staphylococcus aureus* is a pathogen responsible for a wide range of diseases and conditions. Examples of diseases and conditions caused by *S*. *aureus* include bacteremia, infective endocarditis, folliculitis, furuncle, carbuncle, impetigo, bullous impetigo, cellulitis, botryomyosis, toxic shock syndrome, scalded skin syndrome, central nervous system infections, infective and inflammatory eye disease, osteomyletitis and other infections of joints and bones, and respiratory tract infections. (*The Staphylococci in Human Disease,* Crossley and Archer (eds.), Churchill Livingstone Inc. 1997.)

Immunological based strategies can be employed to control *S*. *aureus* infections and the spread of *S*. *aureus.* Immunological based strategies include passive and active immunization. Passive immunization employs immunoglobulins targeting *S*. *aureus.* Active immunization induces immune responses against *S*. *aureus.*

Potential *S*. *aureus* vaccines target *S*. *aureus* polysaccharides and polypeptides. Targeting can be achieved using suitable *S*. *aureus* polysaccharides or polypeptides as vaccine components. Examples of polysaccharides that may be employed as possible vaccine components include *S*. *aureus* type 5 and type 8 capsular polysaccharides. (Shinefield et al., N. Eng. J. Med. 346:491-496, 2002.) Examples of polypeptides that may be employed as possible vaccine components include collagen adhesin, fibrinogen binding proteins, and clumping factor. (Mamo et al., FEMS Immunology and Medical Microbiology 10:47-54, 1994, Nilsson et al., J. Clin. Invest. 101:2640-2649, 1998, Josefsson et al., The Journal of Infectious Diseases 184:1572-1580, 2001.)

Information concerning *S*. *aureus* polypeptide sequences has been obtained from sequencing the *S*. *aureus* genome. (Kuroda et al., Lancet 357:1225-1240, 2001, Baba et al., Lancet 359:1819-1827, 2000, Kunsch *et al.,* European Patent Publication EP 0 786 519, published July 30, 1997.) To some extent bioinformatics has been employed in efforts to characterize polypeptide sequences obtained from genome sequencing. (Kunsch *et al.,* European Patent Publication EP 0 786 519, published July 30, 1997.)

Techniques such as those involving display technology and sera from infected patients have been used in an effort to identify genes coding for potential antigens. (Foster *et al.,* International Publication Number WO 01/98499, published December 27, 2001, Meinke *et al.,* International Publication Number WO 02/059148, published August 1, 2002, Etz et al., PNAS 99:6573-6578, 2002.)

WO-A-03011899 (University of Sheffield and Biosynexus Incorporated) discloses a sequence, AX768656, which differs from SEQ ID NO: 1 in the present application by having two substitutions and over 50 additional amino acids at the amino terminus and over 30 additional amino acids at the carboxy terminus.

### SUMMARY OF THE INVENTION

The present invention features polypeptides comprising an amino acid sequence structurally related to SEQ ID NO: 1 and uses of such polypeptides. SEQ ID NO: 1 is a truncated derivative of a full length *S*. *aureus* polypeptide. The full-length polypeptide is referred to herein as full length "sai-1". A His-tagged derivative of SEQ ID NO: 1 was found to produce a protective immune response against *S*. *aureus.*

Reference to "protective" immunity or immune response indicates a detectable level of protection against *S*. *aureus* infection. The level of protection can be assessed using animal models such as those described herein.

Thus, a first aspect of the present invention describes a polypeptide immunogen comprising an amino acid sequence with up to 26 amino acid deletions from, additions to or substitutions of SEQ ID NO: 1, and the polypeptide provides protective immunity against *S*. *aureus.* SEQ ID NO: 7 provides a full length sai-1 polypeptide, wherein amino acids 261-294 provide the carboxyl terminus domain starting at the LPXTG motif

Reference to "immunogen" indicates the ability to produce a protective immune response.

Reference to comprising an amino acid sequence with up to 26 amino acid deletions from, additions to or substitutions of SEQ ID NO: 1 indicates that a SEQ ID NO: 1 related region is present and additional polypeptide regions may be present.

Percent identity (also referred to as percent identical) to a reference sequence is determined by aligning the polypeptide sequence with the reference sequence and determining the number of identical amino acids in the corresponding regions. This number is divided by the total number of amino acids in the reference sequence (*e.g*., SEQ ID NO: 1) and then multiplied by 100 and rounded to the nearest whole number.

Another aspect of the present invention describes an immunogen comprising a polypeptide that provides protective immunity against *S*. *aureus.* The immunogen consists of the polypeptide and one or more additional regions or moieties covalently joined to the polypeptide at the carboxyl terminus or amino terminus, wherein each region or moiety is independently selected from a region or moiety having at least one of the following properties: enhances the immune response, facilitates purification, or facilitates polypeptide stability.

Reference to "additional region or moiety" indicates a region or moiety different from a sai-1 region. The additional region or moiety can be, for example, an additional polypeptide region or a non-peptide region.

Another aspect of the present invention describes a composition able to induce protective immunity against *S*. *aureus* in a patient. The composition comprises a pharmaceutically acceptable carrier and an immunologically effective amount of an immunogen that provides protective immunity against *S*. *aureus.*

An immunologically effective amount is an amount sufficient to provide protective immunity against *S*. *aureus* infection. The amount should be sufficient to significantly prevent the likelihood or severity of a *S*. *aureus* infection.

Another aspect of the present invention describes a nucleic acid comprising a recombinant gene encoding a polypeptide that provides protective immunity against *S*. *aureus.* A recombinant gene contains recombinant nucleic acid encoding a polypeptide along with regulatory elements for proper transcription and processing (which may include translational and post translational elements). The recombinant gene can exist independent of a host genome or can be part of a host genome.

A recombinant nucleic acid is nucleic acid that by virtue of its sequence and/or form does not occur in nature. Examples of recombinant nucleic acid include purified nucleic acid, two or more nucleic acid regions combined together that provides a different nucleic acid than found in nature, and the absence of one or more nucleic acid regions (*e.g*., upstream or downstream regions) that are naturally associated with each other.

Another aspect of the present invention describes a recombinant cell. The cell comprises a recombinant gene encoding a polypeptide that provides protective immunity against *S. aureus.*

Another aspect of the present invention describes a method of making a ' Polypeptide that provides protective immunity against *S*. *aureus.* The method involves growing a recombinant cell containing recombinant nucleic acid encoding the polypeptide and purifying the polypeptide.

Another aspect of the present invention describes a polypeptide that provides protective immunity against *S*. *aureus* made by a process comprising the steps of growing the recombinant cell containing recombinant nucleic acid encoding the polypeptide in a host and purifying the polypeptide. Different host cells can be employed:

Also disclosed is method of inducing a protective immune response in a patient against *S*. *aureus.* The method comprises the step of administering to the:patient an immunologically effective amount of an immunogen that provides protective immunity against *S. aureus.*

Unless particular terms are mutually exclusive, reference to "or" indicates either or both possibilities. Occasionally phrases such as "and/or" are used to highlight either or both possibilities.

Reference to open-ended terms such as "comprises" allows for additional elements or steps. Occasionally phrases such as "one or more" are used with or without open-ended terms to highlight the possibility of additional elements or steps.

Unless explicitly stated reference to terms such as "a" or "an" is not limited to one. For example, "a cell" does not exclude "cells". Occasionally phrases such as one or more are used to highlight the possible presence of a plurality.

Other features and advantages of the present invention are apparent from the additional descriptions provided herein including the different examples. The provided examples illustrate different components and methodology useful in practicing the present invention. The examples do not limit the claimed invention. Based on the present disclosure the skilled artisan can identify and employ other components and methodology useful for practicing the presentcticinath6pto a present invention.

### BRIEF DESCRIPTION OF THE DRAWINGS

Figure 1 illustrates the amino acid sequence: of SEQ ID NO: 1,
Figure 2 illustrates the amino acid sequence of SEQ ID NO: 2.

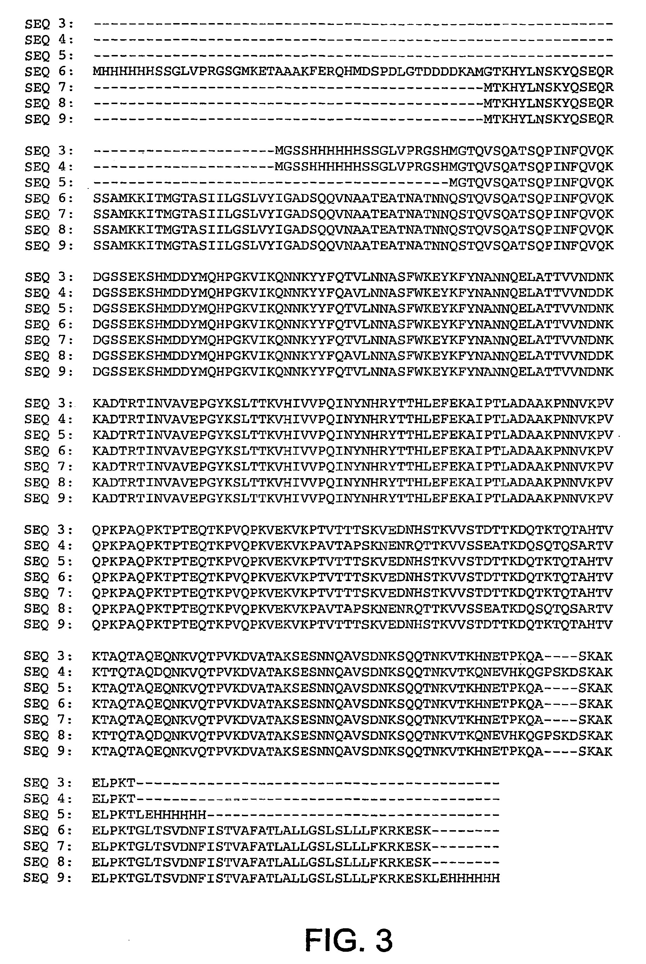

Figure 3 illustrate a sequence comparison between SEQ ID NO: 3 (SEQ 3), SEQ. ID NO: 4 (SEQ 4), SEQ: ID NO: 5 (SEQ 5), SEQ ID NO: 6 (SEQ 6); SEQ ID NO: 7 (SEQ.7), SEQ ID NO: 8 (SEQ 8), and SEQ.ID NO: 9 (SEQ 9): SEQ ID NO:3 is an amino His-tagged construct of SEQ ID NO: 1. SEQ ID NO: 4 is an amino His-tagged construct of SEQ ID:NO:2. SEQ ID NO: 5 is a carboxyl His-tagged construct of SEQ ID NO:1. SEQ ID NO: 6 is an amino His-tagged construct of SEQ ID NO: 7. SEQ ID NO: 7 is full length COL sai-1 sequence. SEQ ID NO: 8 is sai-1 ATTC # AB042826. SEQ ID NO: 9 is a carboxyl His-tagged construct of SEQ ID NO: 7.

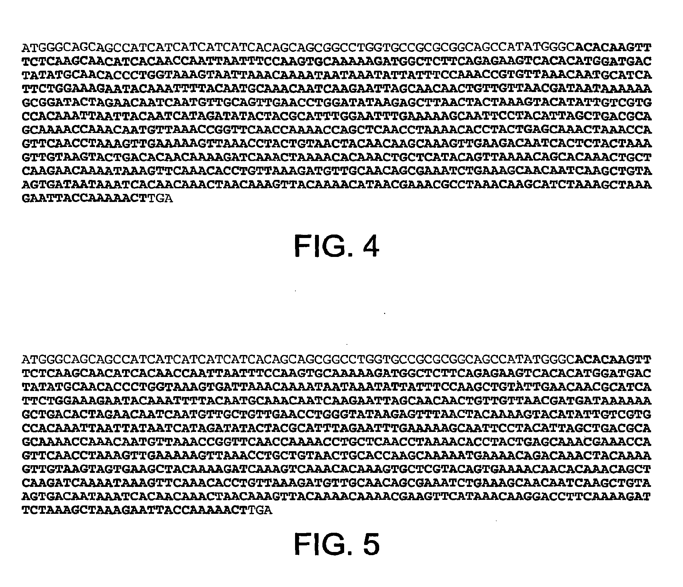

Figure 4 illustrates a nucleic acid sequence encoding SEQ ID NO: 3. The region encoding amino acids 3-260 of SEQ ID NO: 1 is shown in bold.
Figure 5 illustrates a nucleic acid sequence encoding for SEQ ID NO: 4. The region encoding amino acids 3-264 of SEQ ID NO: 2 is shown in bold.

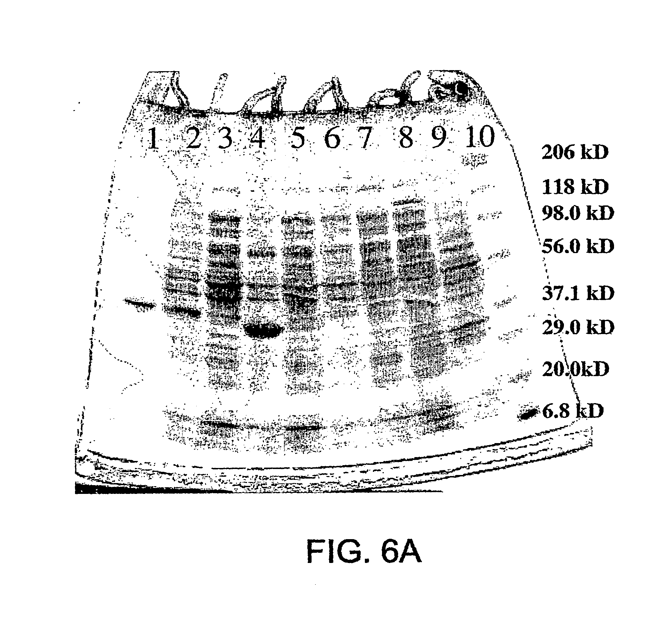

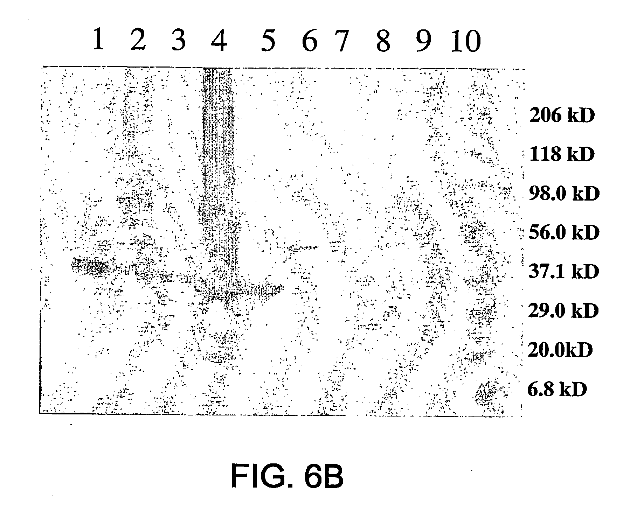

Figures 6A and 6B show an exemplary Coomassie stain of an SDS-PAGE gel and a Western blot, respectively, comparing intracellular expression from nucleic acid encoding SEQ ID NO: 1 related proteins. The Western blot was probed using an anti-his antibody. Lanes- 1, Purified SEQ ID NO: 3 (100 ng); 2, SEQ ID NO: 3 E. *coli* crude lysate (with induction); 3, SEQ ID NO: 3 *E. coli* crude lysate (no induction); 4, SEQ ID NO: 5 *E. coli* crude lysate (with induction); 5, SEQ ID NO: 5 *E. coli* crude lysate (no induction); 6, SEQ ID NO: 6 *E. coli* crude lysate (with induction); 7, SEQ ID NO: 6 *E. coli* crude lysate (no induction); 8, SEQ ID NO: 9 *E*. *coli* crude lysate (with induction); 9, SEQ ID NO: 9 *E. coli* crude lysate (no induction); 10, Standard.
Figures 7A and 7B illustrate survival data from separate experiments using a SEQ ID NO: 3 polypeptide in aluminum hydroxyphosphate adjuvant (AHP). The polypeptide is referred to as "SEQ 3" is Figure 7A and "Vaccine" in Figure 7B.

### DETAILED DESCRIPTION OF THE INVENTION

SEQ ID NO: 1 is a truncated derivative of a *S*. *aureus* transferrin binding protein. An amino His-tagged derivative of SEQ ID NO: 1 was found to be expressed well in *E. coli* and to provide protective immunity against *S*. *aureus* infection. (See Example *infra*.)

SEQ ID NO: 1 was produced based on a full length transferrin binding protein by modifying the encoding nucleic acid to remove the amino signal sequence, to remove a carboxyl hydrophobic region, to add an amino terminus methionine, and to add a restriction site to the amino terminus. The removed hydrophobic region followed a LPXTG motif. The addition of the amino terminus restriction site resulted in a Serine to Glycine substitution.

The ability of polypeptides structurally related to SEQ ID NO: 1 to provide protective immunity is illustrated using a polypeptide of SEQ ID NO: 3. SEQ ID NO: 3 is an amino His-tag derivative of SEQ ID NO: 1. The His-tag facilitates polypeptide purification and identification.

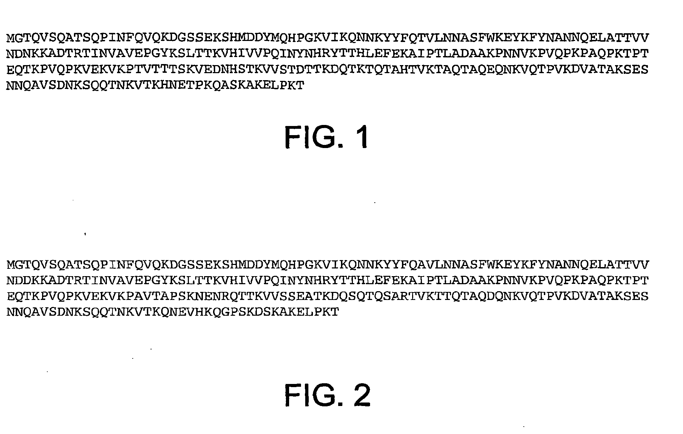

Polypeptides structurally related to SEQ ID NO: 1 include polypeptides containing corresponding regions present in different *S*. *aureus* strains and derivatives of naturally occurring regions. The amino acid sequence of SEQ ID NO: 1 is illustrated in Figure 1. Figure 2 (SEQ ID NO: 2) illustrates an example of a corresponding region found in a different *S*. *aureus* strain modified in a similar manner as SEQ ID NO: 1.

SEQ ID NO: 1 and SEQ ID NO: 2 are based on different naturally occurring full length S. *aureus* sai-1 sequences (SEQ ID NOs: 7 and 8). Figure 3 provides a sequence comparison that includes. SEQ ID NOs: 7 and 8 along with different His-tagged constructs based on SEQ ID NOs: 1,2 and 7.

### Sai-1 Sequences

Sai-1 sequences have been given different designations in different references: Examples of different designations are provided in Kuroda et al, Lancet 357 ; 1225- 1240 , 2001 (SAV1130 and SA0977); Baba et al., Lancet 359:1819-1827, 2002 (MW1012); Mazmanian et al Molecular Microbiology 40(5):1049-1057-; 2001 (SasE); Taylor and Heinrichs Mol. Microbiol. 43(6):1603-1614 (StbA)2 2002; and Mazmanian et al., PNAS 99(4):2293-2298, 2002 and Mazmanian et al., Science 299:906-909, 2003 (IsdA).

A polypeptide sequence corresponding to a sai-1 protein sequence appears to be provided in different patent publications. (Meinke *et al.,* International Publication Number WO 02/059148, published August 1, 2002, Masignani *et al*.,International Publication Number WO 02/094868, published November 28, 2002, Foster *et al*.,International Publication Number WO 02/102829, published December 27,2002, and Foster *et. al*., International Publication Number WO 03/011899, published February 13, 2003.)

Different sai-1 sequences may be present in different strains of S. aureus. Two examples of sai-1 sequences are provided by SEQ ID NO. 7 and 8. Other naturally occurring sai-1 sequences can be identifies based on the presence of a high degree of sequence similarity or contiguous amino acids compared to a known sai-1 sequence. Contiguous amino acids provide characteristic tags. In different embodiments, a naturally occurring sai-1 sequence is a sequence found in *a Staphylococcus,* preferably *S. aureus,* having up to 26 amino acid delections from additions to or substitutions of SEQ ID NO: 1.

Sequence similarity can be determined by different algorithms and techniques well known in the art. Generally, sequence similarity is determined by techniques aligning two sequences to obtain maximum amino acid identity, allowing for gaps, additions and substitutions, in one of the sequences:

Sequence similarity can be determined, for example, using a local alignment tool utilizing the program lalign (developed by Huang and Miller, Adv. Appl. Math .12:337-357, 1991, for the «sim» program). The options and environment variables are:-f # Penalty for the first residue a gap (-14 by default); -g # Penalty for each additional residue in a gap (-4 by default)-s str (SMATRIX) the filename of an alternative scoring matrix file. For protein. sequences, PAM250 is used by default-w # (LINLEN) output line length for sequence alignments (60).

### SEQ ID NO: 1 Related Polypeptides

SEQ ID NO: 1 related polypeptides contain an amino acid sequence having up to 26 amino acid deletions from additions to or subtitutions of SEQ ID NO: 1. Reference to "polypeptide" does not provide a minimum or maximum size limitation.

SEQ ID NO: 2 is an example of a polypeptide structurally related to SEQ ID NO: 1. In different embodiments, the SEQ ID. NO: 1 related polypeptide is at 94%, or at least 99% identical to SEQ ID NO: 1; at least 94% or 99% identical to SEQ ID NO: 2; differs from SEQ ID NO: 1 or SEQ ID NO: 2 by 0,1, 2, 3, 4, 5, 6,7, 8, 9,10, 12, 13, 14, 15, 16; 17,18,19, or 20 amino acid alterations; or consists essentially of amino acids 3-260 of SEQ ID NO: 1 or 3-264 of SEQ ID NO: 2. Each amino acid alteration is independently an addition, deletion or substitution.

Reference to "consists essentially" of indicated amino acids indicates that the referred to amino acids are present and additional amino acids may be present. The additional amino acids can be at the carboxyl or amino terminus. In different embodiments 1, 2,3,4, 5,6, 7, 8, 9, 10,11, 12, 13, 14, 15, 16, 17, 18, 19, or 20 additional amino acids are present. A preferred additional amino acid is an amino terminus methionine.

Alterations can be made to SEQ ID NOs: 1 or 2 to obtain derivatives that can *induce protective immunity agains S. aureus.* Alterations can be performed, for example, to obtain a derivative retaining the ability to induce protective immunity against *S. aureus* or to obtain a derivative that in addition to providing protective immunity also has a region that can achieve a particular purpose.

Figure 2 provides a sequence comparison that includes full length sai-1 sequences (SEQ ID NOs: 7 and 8). The comparison illustrates amino acid differences between *S. aureus* isolates that can be used to guide the design of potential alterations to SEQ ID NO: 1 or 2. In addition, alterations can be made taking into account known properties of amino acids.

Generally, in substituting different amino acids to retain activity it is preferable to exchange amino acids having similar properties. Factors that can be taken into account for an amino acid substitution include amino acid size, charge, polarity, aud hydrophobicity. The effect of different amino acid R-groups on amino acid properties are well known in the art; (See, for examples, Ausubel, Current Protocols in Molecular Biology, John Wiley, 1987-2002, Appendix 1C.)

In exchanging amino acids to maintain activity, the replacement amino acid should have one or more similar properties such as approximately the same charge and/or size and/or polarity and/or hydrophobicity. For example, substituting valine for leucine, arginine for lysine, and asparagine for glutamine are good candidates for not causing a change in polypeptide functioning.

Alterations to achieve a particular purpose include those designed to facilitate production or efficacy of the polypeptide; or cloning of the encoded nucleic acid. Polypeptide production can be facilitated through the use of an initiation codon (e.g., coding for methionine) suitable for recombinant expression. The methionine may be later removed during cellular processing. Cloning can be facilitated by, for example, the introduction of restriction sites which can be accompanied by amino acid additions or changes.

Efficacy of a polypeptide to induce an immune response can be enhanced through epitope enhancement. Epitope enhancement can be performed using different techniques such as those involving alteration of anchor residues to improve peptide affinity for MHC molecules and those increasing affinity of the peptide-MHC complex for a T-cell receptor. (Berzofsky et al., Nature Review 1:209-219, 2001.)

Preferably, the polypeptide is a purified polypeptide. A "purified polypeptide" is present in an environment lacking one or more other polypeptides with which it is naturally associated and/or is represented by at least about 10% of the total protein present. In different embodiments, the purified polypeptide represents at least about 50%, at least about 75%, or at least about 95% of the total protein in a sample or preparation.

In an embodiment, the polypeptide is "substantially purified". A substantially purified polypeptide is present in an environment lacking all, or most, other polypeptides with which the polypeptide is naturally associated. For example, a substantially purified *S*. *aureus* polypeptide is present in an environment lacking all, or most, other *S*. *aureus* polypeptides. An environment can be, for example, a sample or preparation.

Reference to "purified" or "substantially purified" does not require a polypeptide to undergo any purification and may include, for example, a chemically synthesized polypeptide that has not been purified.

Polypeptide stability can be enhanced by modifying the polypeptide carboxyl or amino terminus. Examples of possible modifications include amino terminus protecting groups such as acetyl, propyl, succinyl, benzyl, benzyloxycarbonyl or t-butyloxycarbonyl; and carboxyl terminus protecting groups such as amide, methylamide, and ethylamide.

Polypeptide purification can be enhanced by adding a group to the carboxyl or amino terminus to facilitate purification. Examples of groups that can be used to facilitate purification include polypeptides providing affinity tags. Examples of affinity tags include a six-histidine tag, trpE, glutathione and maltose-binding protein.

The ability of a polypeptide to produce an immune response can be enhanced using groups that generally enhance an immune response. Examples of groups that can be joined to a polypeptide to enhance an immune response against the polypeptide include cytokines such as IL-2. (Buchan et al., 2000. Molecular Immunology 37:545-552.)

### Polypeptide Production

Polypeptides can be produced using standard techniques including those involving chemical synthesis and those involving purification from a cell producing the polypeptide. Techniques for chemical synthesis of polypeptides are well known in the art. (See *e.g*., Vincent, Peptide and Protein Drug Delivery, New York, N.Y., Decker, 1990.)

Techniques for polypeptide purification from a cell are illustrated in the Example provided below. Additional examples of purification techniques are well known in the art. (See for example, Ausubel, Current Protocols in Molecular Biology, John Wiley, 1987-2002.)

Obtaining polypeptides from a cell is facilitated using recombinant nucleic acid techniques to produce the polypeptide. Recombinant nucleic acid techniques for producing a polypeptide involve introducing, or producing, a recombinant gene encoding the polypeptide in a cell and expressing the polypeptide.

A recombinant gene contains nucleic acid encoding a polypeptide along with regulatory elements for polypeptide expression. The recombinant gene can be present in a cellular genome or can be part of an expression vector.

The regulatory elements that may be present as part of a recombinant gene include those naturally associated with the polypeptide encoding sequence and exogenous regulatory elements not naturally associated with the polypeptide encoding sequence. Exogenous regulatory elements such as an exogenous promoter can be useful for expressing a recombinant gene in a particular host or increasing the level of expression. Generally, the regulatory elements that are present in a recombinant gene include a transcriptional promoter, a ribosome binding site, a terminator, and an optionally present operator. A preferred element for processing in eukaryotic cells is a polyadenylation signal.

Expression of a recombinant gene in a cell is facilitated through the use of an expression vector. Preferably, an expression vector in addition to a recombinant gene also contains an origin of replication for autonomous replication in a host cell, a selectable marker, a limited number of useful restriction enzyme sites, and a potential for high copy number. Examples of expression vectors are cloning vectors, modified cloning vectors, specifically designed plasmids and viruses.

Due to the degeneracy of the genetic code, a large number of different encoding nucleic acid sequences can be used to code for a particular polypeptide. The degeneracy of the genetic code arises because almost all amino acids are encoded by different combinations of nucleotide triplets or "codons". Amino acids are encoded by codons as follows: A=Ala=Alanine: codons GCA, GCC, GCG, GCU
C=Cys=Cysteine: codons UGC, UGU
D=Asp=Aspartic acid: codons GAC, GAU
E=Glu=Glutamic acid: codons GAA, GAG
F=Phe=Phenylalanine: codons UUC, UUU
G=Gly=Glycine: codons GGA, GGC, GGG, GGU
H=His=Histidine: codons CAC, CAU
I=Ile=Isoleucine: codons AUA, AUC, AUU
K=Lys=Lysine: codons AAA, AAG
L=Leu=Leucine: codons UUA, UUG, CUA, CUC, CUG, CUU
M=Met=Methionine: codon AUG
N=Asn=Asparagine: codons AAC, AAU
P=Pro=Proline: codons CCA, CCC, CCG, CCU
Q=Gln=Glutamine: codons CAA, CAG
R=Arg=Arginine: codons AGA, AGG, CGA, CGC, CGG, CGU
S=Ser=Serine: codons AGC, AGU, UCA, UCC, UCG, UCU
T=Thr=Threonine: codons ACA, ACC, ACG, ACU
V=Val=Valine: codons GUA, GUC, GUG, GUU
W=Trp=Tryptophan: codon UGG
Y=Tyr=Tyrosine: codons UAC, UAU

Suitable cells for recombinant nucleic acid expression of sai-1 related polypeptides are prokaryotes and eukaryotes. Examples of prokaryotic cells include *E. coli;* members of the *Staphylococcus* genus, such as *S*. *aureus;* members of the *Lactobacillus* genus, such as *L. plantarum;* members of the *Lactococcus* genus, such as *L. lactis;* and members of the *Bacillus* genus, such as *B. subtilis.* Examples of eukaryotic cells include mammalian cells; insect cells; yeast cells such as members of the *Saccharomyces* genus (*e.g*., *S. cerevisiae*), members of the *Pichia* genus (*e.g., P. pastoris*), members of the *Hansenula* genus (*e.g., H. polymorpha*), members of the *Kluyveromyces* genus (*e.g., K. lactis* or *K. fragilis*) and members of the *Schizosaccharomyces* genus (*e.g., S. pombe*).

Techniques for recombinant gene production, introduction into a cell, and recombinant gene expression are well known in the art. Examples of such techniques are provided in references such as Ausubel, Current Protocols in Molecular Biology, John Wiley, 1987-2002, and Sambrook et al., Molecular Cloning, A Laboratory Manual, 2nd Edition, Cold Spring Harbor Laboratory Press, 1989.

If desired, expression in a particular host can be enhanced through codon optimization. Codon optimization includes use of more preferred codons. Techniques for codon optimization in different hosts are well known in the art.

Depending upon the host used for expression, sai-1 related polypeptides may contain post translational modifications. Reference to "polypeptide" or an "amino acid" sequence of a polypeptide includes polypeptides containing one or more amino acids having a structure of a post-translational modification from a host cell, such as a yeast host.

For example, in *S*. *cerevisiae,* the nature of the penultimate amino acid appears to determine whether the N-terminal methionine is removed. Furthermore, the nature of the penultimate amino acid also determines whether the N-terminal amino acid is N^{α}-acetylated (Huang et al., Biochemistry 26: (1987), 8242-8246, 1987). Thus, within the scope of this invention, the sai-1 related polypeptide may have an N^{α}-acetylated N-terminus and the N-terminal methionine may be removed, depending on which amino acid is in the penultimate position.

In addition, if the sai-1 related polypeptide is targeted for secretion due to the presence of a secretory leader (*e.g*., signal peptide), the protein may be modified by N-linked or O-linked glycosylation. (Kukuruzinska et al., Ann. Rev. Biochem. 56:915-944, 1987.)

### Adjuvants

Adjuvants are substances that can assist an immunogen in producing an immune response. Adjuvants can function by different mechanisms such as one or more of the following: increasing the antigen biologic or immunologic half-life; improving antigen delivery to antigen-presenting cells; improving antigen processing and presentation by antigen-presenting cells; and inducing production of immunomodulatory cytokines. (Vogel, Clinical Infectious Diseases 30(suppl. 3):S266-270, 2000.)

A variety of different types of adjuvants can be employed to assist in the production of an immune response. Examples of particular adjuvants include aluminum hydroxide, aluminum phosphate, or other salts of aluminum, calcium phosphate, DNA CpG motifs, monophosphoryl lipid A, cholera toxin, *E*. *coli* heat-labile toxin, pertussis toxin, muramyl dipeptide, Freund's incomplete adjuvant, MF59, SAF, immunostimulatory complexes, liposomes, biodegradable microspheres, saponins, nonionic block copolymers, muramyl peptide analogues, polyphosphazene, synthetic polynucleotides, IFN-γ, IL-2 and IL-12. (Vogel Clinical Infectious Diseases 30(suppl 3):S266-270, 2000, Klein et al., Journal of Pharmaceutical Sciences 89:311-321, 2000.)

### Patients For Inducing Protective Immunity

A "patient" refers to a mammal capable of being infected with *S*. *aureus.* A patient can be treated prophylactically or therapeutically. Prophylactic treatment provides sufficient protective immunity to reduce the likelihood, or severity, of a *S*. *aureus* infection. Therapeutic treatment can be performed to reduce the severity of a *S*. *aureus* infection.

Prophylactic treatment can be performed using a vaccine containing an immunogen described herein. Such treatment is preferably performed on a human. Vaccines can be administered to the general population or to those persons at an increased risk of *S*. *aureus* infection.

Persons with an increased risk of *S. aureus* infection include health care workers; hospital patients; patients with a weakened immune system; patients undergoing surgery; patients receiving foreign body implants, such a catheter or a vascular device; patients facing therapy leading to a weakened immunity; and persons in professions having an increased risk of burn or wound injury. (The Staphylococci in Human Disease, Crossley and Archer (ed.), Churchill Livingstone Inc. 1997.)

Non-human patients that can be infected with *S. aureus* include cows, pigs, sheep, goats, rabbits, horses, dogs, cats and mice. Treatment of non-human patients is useful in protecting pets and livestock, and in evaluating the efficacy of a particular treatment.

### Combination Vaccines

SEQ ID NO: 1 related polypeptides can be used alone, or in combination with other immunogens, to induce an immune response. Additional immunogens that may be present include: one or more additional *S*. *aureus* immunogens, such as those referenced in the Background of the Invention *supra;* one or more immunogens targeting one or more other *Staphylococcus* organisms such as *S*. *epidermidis, S. haemolyticus, S. warneri,* or *S.lugunnensis;* and one or more immunogens targeting other infections organisms.

### Animal Model System

An animal model system was used to evaluate the efficacy of an immunogen to produce a protective immune response against *Staphylococcus.* Two obstacles encountered in setting up a protective animal model were: (1) very high challenge dose needed to overcome innate immunity and (2) death rate too fast to detect a protective response. Specifically, after bacterial challenge mice succumbed to infection within 24 hours which did not provide sufficient time for the specific immune responses to resolve the infection. If the dose was lowered both control and immunized mice survived the infection.

These obstacles were addressed by developing a slow kinetics lethality model involving *S*. *aureus* prepared from cells in stationary phase, appropriately titrated, and intravenously administered. This slow kinetics of death provides sufficient time for the specific immune defense to fight off the bacterial infection (*e.g*., 10 days rather 24 hours).

*S. aureus* cells in stationary phase can be obtained from cells grown on solid medium. They can also be obtained from liquid, however the results with cells grown on solid media were more reproducible. Cells can conveniently be grown overnight on solid medium. For example, *S*. *aureus* can be grown from about 18 to about 24 hours under conditions where the doubling time is about 20-30 minutes.

*S. aureus* can be isolated from solid or liquid medium using standard techniques to maintain *Staphylococcus* potency. Isolated *Staphylococcus* can be stored, for example, at - 70°C as a washed high density suspension (> 10⁹ colony forming units (CFU)/mL) in phosphate buffered saline containing glycerol.

The *S*. *aureus* challenge should have a potency providing about 80 to 90% death in an animal model over a period of about 7 to 10 days starting on the first or second day. Titration experiments can be performed using animal models to monitor the potency of the stored *Staphylococcus* inoculum. The titration experiments can be performed about one to two weeks prior to an inoculation experiment.

Initial potency for titration experiments can be based on previous experiments. For *S*. *aureus* and the animal model strain Becker a suitable potency was generally found in the range of 5 x 10⁸ to 8 x 10⁸ CFU/ml.

### Administration

Immunogens can be formulated and administered to a patient using the guidance provided herein along with techniques well known in the art. Guidelines for pharmaceutical administration in general are provided in, for example, Vaccines Eds. Plotkin and Orenstein, W.B. Sanders Company, 1999; Remington's Pharmaceutical Sciences 20th Edition, Ed. Gennaro, Mack Publishing, 2000; and Modem Pharmaceutics 2nd Edition, Eds. Banker and Rhodes, Marcel Dekker, Inc., 1990, each of which are hereby incorporated by reference herein.

Pharmaceutically acceptable carriers facilitate storage and administration of an immunogen to a patient. Pharmaceutically acceptable carriers may contain different components such as a buffer, sterile water for injection, normal saline or phosphate buffered saline, sucrose, histidine, salts and polysorbate.

Immunogens can be administered by different routes such as subcutaneous, intramuscular, or mucosal. Subcutaneous and intramuscular administration can be performed using, for example, needles or jet-injectors.

Suitable dosing regimens are preferably determined taking into account factors well known in the art including age, weight, sex and medical condition of the patient; the route of administration; the desired effect; and the particular compound employed. The immunogen can be used in multi-dose vaccine formats. It is expected that a dose would consist of the range of 1.0 µg to 1.0 mg total polypeptide, in different embodiments of the present invention the range is 0.01 mg to 1.0 mg and 0.1 mg to 1.0 mg.

The timing of doses depends upon factors well known in the art. After the initial administration one or more booster doses may subsequently be administered to maintain or boost antibody titers. An example of a dosing regime would be day 1,1 month, a third dose at either 4, 6 or 12 months, and additional booster doses at distant times as needed.

### Generation of Antibodies

A SEQ ID NO: 1 related polypeptide can be used to generate antibodies and antibody fragments that bind to the polypeptide or to *S*. *aureus.* Such antibodies and antibody fragments have different uses including use in polypeptide purification, *S*. *aureus* identification, or in therapeutic or prophylactic treatment against *S*. *aureus* infection.

Antibodies can be polyclonal or monoclonal. Techniques for producing and using antibodies are well known in the art. Examples of such techniques are described in Ausubel, Current Protocols in Molecular Biology, John Wiley, 1987-2002, Harlow et al., Antibodies, A Laboratory Manual, Cold Spring Harbor Laboratory, 1988, and Kohler et al., Nature 256:495-497, 1975.

### EXAMPLES

Examples are provided below further illustrating different features of the present invention. The examples also illustrate useful methodology for practicing the invention. These examples do not limit the claimed invention.

### Example 1: Use of SEQ ID NO: 3 to Provide Protective Immunity

This example illustrates the ability of SEQ ID NO: 1 related polypeptides to provide protective immunity in a model. SEQ ID NO: 3, a His-tagged derivative of SEQ ID NO: 1, was used to provide protective immunity.

### Sai-1 Cloning and Expression

A sai-1 DNA sequence was translated using Vector NTI software and the resulting 355 amino acid sequence was analyzed. PCR primers were designed to amplify the gene starting at the first asparagine residue and ending prior to the stop codon at the terminal asparagine residue. These PCR primers also had additional NcoI (forward primer) and XhoI (reverse primer) sites to facilitate cloning into the expression vector.

The protein was designed to be expressed from the pET28 vector with the terminal His residues and the stop codon encoded by the vector. In addition, a glycine residue was added to the protein after the methionine initiator for Sai-N. Sai-C has a carboxyl terminal His-tag and is composed of the 350 amino acid sai-1 protein and an additional 8 amino acids at the carboxyl end comprising the poly His tail and vector sequences. Sai-N had an amino terminal His-tag and is composed of a 351 amino acid sai-1 protein (includes the glycine insert), with an additional 46 amino acids comprising the poly His tail and vector sequences.

PCR amplified sequences were ligated into the pET28 vector (Novagen) using the NcoI/XhoI sites that had been engineered into the PCR primers and introduced into *E. coli* DH5α (Invitrogen) by heat shock. PCR amplified sequences were ligated into the pET28 vector (Novagen) using the NcoI/XhoI sites that had been engineered into the PCR primers and introduced into *E*. *coli* DH5α (Invitrogen) by heat shock. Colonies were selected, grown in LB with 30 µg/mL kanamycin, DNA minipreps made (Promega), and insert integrity determined by restriction digestion and PCR. Four minipreps with correct insert size were sequenced using the primers listed in Table 1. A clone was selected containing no DNA changes from the desired sequence.

**Table 1**

| SEQ ID NO: | Description | Sequence |
|---|---|---|
| 12 | Sail-CF | GAGATATACCATGGGCACAAAACATTATTTAAACAGT |
| 13 | Sail-CR | CCGGCGGCCCTCGAGTTTAGATTCTTTTCTTTTGAA |
| 14 | Sail-NF | GAGATATACCATGGGCACAAAACATTATTTAAACAGT |
| 15 | Sail-NR | CCGGCGGCCCTCGAGTTATTTAGATTCTTTTCTTTTGAA |
| 16 | Sail-C2F | GAGATATACCATGGGCACACAAGTTTCTCAAGCAACATCAC |
| 17 | Sail-C2R | GGTGGTGCTCGAGAGTTTTTGGTAATTCTTTAGCTT |
| 18 | Sail-N2F | GAGATATCATATGGGCACACAAGTTTCTCAAGCAACATCAC |
| 19 | Sail-N2R | GGTGGTGCTCGAGTCAAGTTTTTGGTAATTCTTTAGCTT |

*E. coli* HMS174(DE3) cells (Novagen) were transformed and grown on LB plates containing kanamycin (30ug/ml); 3 colonies were selected for expression testing. Liquid LB (kanamycin) cultures were incubated at 37°C, 250 rpm until the A₆₀₀ was between 0.6 and 1.0 and then induced by the addition of IPTG to final concentrations of 1 mM followed by three hours further incubation. Cultures were harvested by centrifugation at 5000 x g for 5 minutes at 4°C. Cells were resuspended in 500 µl lysis buffer (Bug Buster, with protease inhibitors, Novagen). An equal volume of loading buffer (supplemented with β-mecapto ethanol to 5% final volume) was added prior to heating the samples at 70°C for 5 minutes. Extracts were run on Novex 4-20% Tris-Glycine gels and assayed for protein (Coomassie Blue stained) and blotted onto nitrocellulose and probed with anti-HIS6 antibodies (Zymedd). The expression observed was extremely low.

The protein was re-analyzed; a putative signal sequence was removed as was the down stream region from the LPXTG motif. These PCR primers also had additional NdeI (forward primer) and XhoI (reverse primer) sites to facilitate cloning into the expression vector.

The protein was designed to be expressed from the pET28 vector with the terminal His residues and the stop codon encoded by the vector. In addition, a glycine residue was added to the protein after the methionine initiator. Sai-N2 (SEQ ID NO: 3) contains an amino His-tag. Sai-C2 (SEQ ID NO: 5) contains a carboxyl terminal His-tag.

PCR amplified sequences were ligated into the pET28 vector (Novagen) using the NdeI/XhoI sites that had been engineered into the PCR primers and introduced into *E*. *coli* DH5α (Invitrogen) by heat shock. PCR amplified sequences were ligated into the pET28 vector (Novagen) using the NdeI/XhoI sites that had been engineered into the PCR primers and introduced into *E*. *coli* DH5α (Invitrogen) by heat shock. Colonies were selected, grown in LB with 30 µg/mL kanamycin, DNA minipreps made (Promega), and insert integrity determined by restriction digestion and PCR. Four minipreps with correct insert size were sequenced using the primers listed in Table 1. A clone was selected containing no DNA changes from the desired sequence.

*E. coli* HMS174(DE3) cells (Novagen) were transformed and grown on LB plates containing kanamycin (30ug/ml); 3 colonies were selected for expression testing. Liquid LB (kanamycin) cultures were incubated at 37°C, 250 rpm until the A₆₀₀ was between 0.6 and 1.0 and then induced by the addition of IPTG to final concentrations of 1 mM followed by three hours further incubation. Cultures were harvested by centrifugation at 5000 x g for 5 minutes at 4°C. Cells were resuspended in 500 µl lysis buffer (Bug Buster, with protease inhibitors, Novagen). An equal volume of loading buffer (supplemented with β-mecapto ethanol to 5% final volume) was added prior to heating the samples at 70°C for 5 minutes. Extracts were run on Novex 4-20% Tris-Glycine gels and assayed for protein (Coomassie Blue stained) and blotted onto nitrocellulose and probed with anti-HIS6 antibodies (Zymedd).

### SEQ ID NO: 3 Purification

Recombinant *E. coli* cells (46 grams wet cell weight) were suspended in Lysis Buffer (50 mM sodium phosphate, pH 8.0, 0.15 M NaCl, 2 mM MgCl₂, 10 mM imidazole, 0.1 % Tween^{™}-80, and 0.02% sodium azide) at 3 ml per gram of cell wet weight. Protease Inhibitor Cocktail for use with poly-(Histidine)-tagged proteins (Roche #1873580) was added to the suspension at 1 tablet per 15 grams of cell paste. Benzonase^{™} (EM Ind.) was added to 1 µL/mL. Cell lysis was accomplished by passing the suspension through a microfluidizer at 14,000 PSI (Microfluidics Model 110S) three times. The cell suspension was cooled on ice between each pass so that the temperature remained below 25°C. Cell debris was pelleted at 11,000 x g for 30 minutes at 4°C, and the supernatant retained.

Proteins bearing a His-tag were purified from the supernatant. The supernatant was mixed with 12 mL of Ni⁺-NTA agarose (Qiagen) at 4°C with gentle inversion for 18 hours. The mixture was poured into an open column (1.5 cm x 20 cm) and the non-bound fraction was collected in bulk. The column was washed with Wash Buffer (50 mM sodium phosphate, pH 8.0, 0.3 M NaCl, 20 mM imidazole, and 0.1% Tween™-80). His-tagged protein was eluted with a step gradient of 300 mM imidazole, 20 mM Tris-HCl, pH 8, 0.3 M NaCl, 0.1% Tween™-80.

Fractions containing SEQ ID NO: 3 polypeptide were detected by Coomassie stained SDS-PAGE and pooled. Pooled fractions were filtered through a 0.2 micron filter to remove particulate material, and were applied on a size-exclusion column (Sephacryl S-300 26/60 column, Amersham Biosciences) and eluted at 1 mL/min with 30 mM MOPS pH 7.0, 0.3 M NaCl, and 10% glycerol. Fractions containing SEQ ID NO: 3 polypeptide were detected by Coomassie stained SDS-PAGE and Western blotting (anti-tetra His Mab, Qiagen). Protein was determined by BCA (Pierce). Purity was determined by densitometry of Coomassie stained gels.

### Preparation of S. aureus Challenge

S. aureus was grown on TSA plates at 37°C overnight. The bacteria were washed from the TSA plates by adding 5 ml of PBS onto a plate and gently resuspending the bacteria with a sterile spreader. The bacterial suspension was spun at 6000 rpm for 20 minutes using a Sorvall RC-5B centrifuge (DuPont Instruments). The pellet was resuspended in 16% glycerol and aliquots were stored frozen at -70°C.

Prior to use, inocula were thawed, appropriately diluted and used for infection. Each stock was titrated at least 3 times to determine the appropriate dose inducing slow kinetics of death in naive mice. The potency of the bacterial inoculum (80 to 90% lethality) was constantly monitored to assure reproducibility of the model. Ten days before each challenge experiment, a group of 10 control animals (immunized with adjuvant alone) were challenged and monitored.

### Protection Studies for a SEQ ID NO: 3 Polypeptide

Twenty BALB/c mice were immunized with three doses of a SEQ ID NO: 3 polypeptide (20 µg per dose) on aluminum hydroxyphosphate adjuvant (450 µg per dose). Aluminum hydroxyphosphate adjuvant (AHP) is described by Klein et al., Journal of Pharmaceutical Sciences 89, 311-321, 2000*.* The doses were administered as two 50 µl injections on days 0, 7 and 21. The mice were bled on day 28, and their sera were screened by ELSIA for reactivity to the SEQ ID NO: 3 polypeptide.

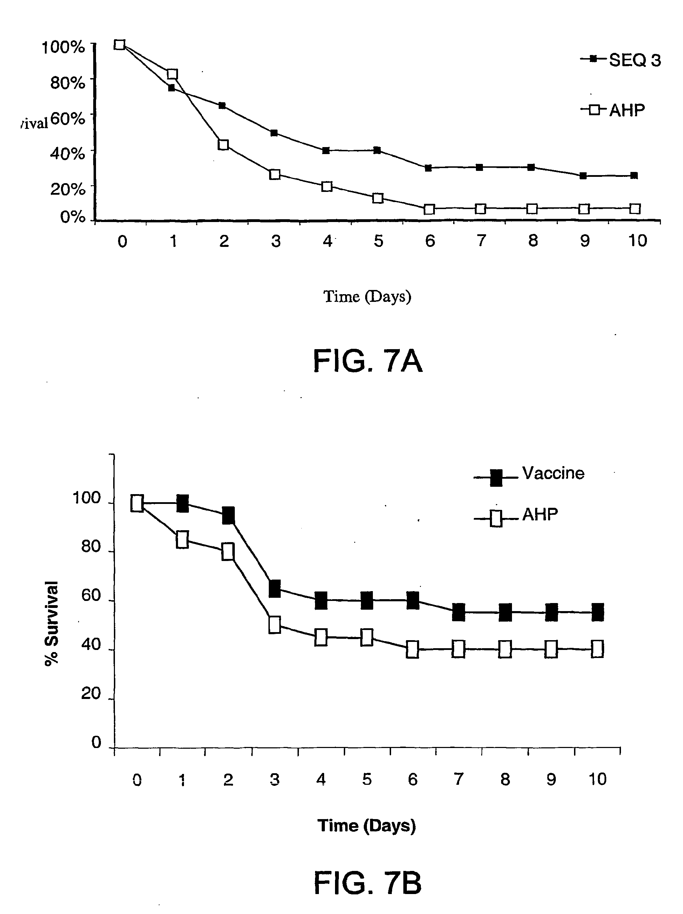

On day 35 of the experiment the mice were challenged by intravenous injection of *S. aureus* grown at a dose (8.0 x 10⁸ CFU ml). The mice were monitored over a 10 day period for survival. At the end of the experiment 5 mice survived the SEQ ID NO: 3 polypeptide immunized group, compared to 2 surviving in the AHP control group containing 30 mice. The experiment was repeated using 20 immunized mice and 20 control mice. Results for both experiments are shown in Figure 7A and 7B.

### Example 2: Intracellular Expression From Nucleic Acid Encoding SEO ID NO: 1 Related Proteins

Figures 6A and 6B show an exemplary Coomassie stain of an SDS-PAGE gel and a Western blot, respectively, comparing intracellular expression from nucleic acid encoding SEQ ID NO: 1 related proteins. The Western blot was probed using an anti-his antibody. Lanes- 1, Purified SEQ ID NO: 3 (100 ng); 2, SEQ ID NO: 3 *E. coli* crude lysate (with induction); 3, SEQ ID NO: 3 *E*. *coli* crude lysate (no induction); 4, SEQ ID NO: 5 *E*. *coli* crude lysate (with induction); 5, SEQ ID NO: 5 *E. coli* crude lysate (no induction); 6, SEQ ID NO: 6 *E*. *coli* crude lysate (with induction); 7, SEQ ID NO: 6 *E*. *coli* crude lysate (no induction); 8, SEQ. ID NO: 9 *E*. *coli* crude lysate (with induction); 9, SEQ.ID NO: 9 *E. coli* crude lysate (no induction); 10, Standard.

## Claims

1. A polypeptide immunogen consisting of an amino acid sequence with up to 26 amino acid deletions from, additions to or substitutions of SEQ ID NO: 1, wherein said polypeptide immunogen provides protective immunity against *S*. *aureus.*

2. The polypeptide immunogen of claim 1, wherein said polypeptide immunogen consists of an amino acid sequence at least 94% identical to either SEQ ID NO: 1 or SEQ ID NO: 2.

3. The polypeptide immunogen of claim 1, wherein said polypeptide immunogen is at least 99% identical to SEQ ID NO: 1.

4. The polypeptide immunogen of claim 1, wherein said polypeptide immunogen consists of an amino acid sequence with up to 10 amino acid alterations from SEQ ID NO: 1.

5. The polypeptide immunogen of claim 1, wherein said polypeptide immunogen consists of an amino acid sequence with up to 5 amino acid alterations from SEQ ID NO: 1.

6. The polypeptide immunogen of claim 5, wherein said polypeptide immunogen consists of an amino acid sequence with up to 1 amino acid alteration from SEQ ID NO: 1.

7. The polypeptide immunogen of claim 1, wherein said polypeptide immunogen consists essentially of amino acids 3-260 of SEQ ID NO: 1 or 3-264 of SEQ NO: 2.

8. The polypeptide immunogen of claim 1, wherein said polypeptide immunogen consists of an amino acid sequence of SEQ ID NO: 1.

9. The polypeptide immunogen of any one of claims 1-8, wherein said polypeptide immunogen is substantially purified.

10. An immunogen comprising the polypeptide immunogen of any one of claims 1-9, wherein said immunogen consists of said polypeptide immunogen and one or more additional regions or moieties covalently joined to said polypeptide immunogen at the carboxyl terminus or amino terminus, wherein each region or moiety is independently selected from a region or moiety having at least one of the following properties: enhances the immune response, facilitates purification, or facilitates polypeptide stability.

11. A composition able to induce a protective immune response in a patient comprising an immunologically effective amount of the immunogen of any one of claims 1-10 and a pharmaceutically acceptable carrier.

12. The composition of claim 11, wherein said composition further comprises an adjuvant.

13. A nucleic acid comprising a recombinant gene comprising a nucleotide sequence encoding the polypeptide of any one of claims 1-8.

14. The nucleic acid of claim 13, wherein said nucleic acid is an expression vector.

15. A recombinant cell comprising a recombinant gene comprising a nucleotide sequence encoding the polypeptide of any one of claims 1-8.

16. A method of making a *S*. *aureus* polypeptide that provides protective immunity comprising the steps of:
(a) growing the recombinant cell of claim 1 under conditions wherein a polypeptide is expressed; and
(b) purifying said polypeptide.

17. A polypeptide immunogen of any one of claims 1-8 for use in a method of treatment by therapy.

18. A polypeptide immunogen for use of claim 17 wherein the therapy is the prophylactic treatment of *S*. *aureus* infection.

19. The polypeptide immunogen for use of claim 18 wherein the therapy is in a human.

20. Use of a polypeptide immunogen of any one of claims 1-8 for the manufacture of a medicament for the prophylactic treatment of *S*. *aureus* infection.

21. Use according to claim 20 wherein the prophylactic treatment is in a human.

22. A combination of a polypeptide immunogen of any one of claims 1 to 9 and another immunogen.

## Patentansprüche

1. Ein Polypeptid-Immunogen, bestehend aus einer Aminosäuresequenz mit bis zu 26 Aminosäure-Deletionen, -Ergänzungen oder -Substitutionen von bzw. zu SEQ ID NO: 1, wobei das Polypeptid-Immunogen schützende Immunität gegen *S*. *aureus* verleiht.

2. Das Polypeptid-Immunogen nach Anspruch 1, wobei das Polypeptid-Immunogen aus einer Aminosäuresequenz besteht, die zu wenigstens 94% identisch ist mit entweder SEQ ID NO: 1 oder SEQ ID NO: 2.

3. Das Polypeptid-Immunogen nach Anspruch 1, wobei das Polypeptid-Immunogen zu wenigstens 99% identisch ist mit SEQ ID NO: 1.

4. Das Polypeptid-Immunogen nach Anspruch 1, wobei das Polypeptid-Immunogen aus einer Aminosäuresequenz mit bis zu 10 Aminosäure-Abänderungen von SEQ ID NO: 1 besteht.

5. Das Polypeptid-Immunogen nach Anspruch 1, wobei das Polypeptid-Immunogen aus einer Aminosäuresequenz mit bis zu 5 Aminosäure-Abänderungen von SEQ ID NO: 1 besteht.

6. Das Polypeptid-Immunogen nach Anspruch 5, wobei das Polypeptid-Immunogen aus einer Aminosäuresequenz mit bis zu 1 Aminosäure-Abänderung von SEQ ID NO: 1 besteht.

7. Das Polypeptid-Immunogen nach Anspruch 1, wobei das Polypeptid-Immunogen im Wesentlichen aus den Aminosäuren 3-260 von SEQ ID NO: 1 oder 3-264 von SEQ ID NO: 2 besteht.

8. Das Polypeptid-Immunogen nach Anspruch 1, wobei das Polypeptid-Immunogen aus einer Aminosäuresequenz von SEQ ID NO: 1 besteht.

9. Das Polypeptid-Immunogen nach einem der Ansprüche 1-8, wobei das Polypeptid-Immunogen im Wesentlich gereinigt ist.

10. Ein Immunogen, umfassend das Polypeptid-Immunogen nach einem der Ansprüche 1-9, wobei das Immunogen aus dem Polypeptid-Immunogen und einer oder mehreren zusätzlichen Regionen oder Gruppen besteht, die kovalent am Carboxylende oder am Aminoende an das Polypeptid-Immunogen gebunden sind, wobei jede Region oder Gruppe unabhängig ausgewählt ist aus einer Region oder Gruppe mit wenigstens einer der folgenden Eigenschaften: verstärkt die Immunreaktion, unterstützt die Reinigung oder fördert die Polypeptidstabilität.

11. Eine Zusammensetzung, die in der Lage ist, eine schützende Immunreaktion in einem Patienten hervorzurufen, umfassend eine immunologisch wirksame Menge des Immunogens nach einem der Ansprüche 1-10 und einen pharmazeutisch wirksamen Träger.

12. Die Zusammensetzung nach Anspruch 11, wobei die Zusammensetzung ferner einen Hilfsstoff umfasst.

13. Eine Nukleinsäure, umfassend ein rekombinantes Gen, das eine Nukleotidsequenz umfasst, die für das Polypeptid nach einem der Ansprüche 1-8 kodiert.

14. Die Nukleinsäure nach Anspruch 13, wobei die Nukleinsäure ein Expressionsvektor ist.

15. Eine rekombinante Zelle, umfassend ein rekombinantes Gen, das eine Nukleotidsequenz umfasst, die für das Polypeptid nach einem der Ansprüche 1-8 kodiert.

16. Ein Verfahren zur Herstellung eines *S*.-*aureus*-Polypeptids, das schützende Immunität verleiht, umfassend die Schritte:
(a) Kultivieren der rekombinanten Zelle nach Anspruch 15 unter Bedingungen, bei denen ein Polypeptid exprimiert wird, und
(b) Reinigen des Polypeptids.

17. Ein Polypeptid-Immunogen nach einem der Ansprüche 1-8 zur Verwendung bei einem Verfahren zur Behandlung durch Therapie.

18. Ein Polypeptid-Immunogen zur Verwendung nach Anspruch 17, wobei die Therapie die prophylaktische Behandlung einer *S*.-*aureus*-Infektion ist.

19. Das Polypeptid-Immunogen zur Verwendung nach Anspruch 18, wobei die Therapie bei einem Menschen erfolgt.

20. Verwendung eines Polypeptid-Immunogens nach einem der Ansprüche 1-8 zur Herstellung eines Medikaments zur prophylaktischen Behandlung einer *S*.-*aureus*-Infektion.

21. Verwendung gemäß Anspruch 20, wobei die prophylaktische Behandlung bei einem Menschen erfolgt.

22. Eine Kombination aus einem Polypeptid-Immunogen nach einem der Ansprüche 1 bis 9 und einem weiteren Immunogen.

## Revendications

1. Polypeptide immunogène consistant en une séquence d'acides aminés avec jusqu'à 26 délétions, additions ou substitutions des acides aminés de la SEQ ID NO: 1, où ledit polypeptide immunogène fournit une immunité protectrice contre *S. aureus.*

2. Polypeptide immunogène selon la revendication 1, où ledit polypeptide immunogène consiste en une séquence d'acides aminés identique à 94% au moins à la SEQ ID NO: 1 ou à la SEQ ID: 2.

3. Polypeptide immunogène selon la revendication 1, où ledit polypeptide immunogène est identique à 99% au moins à la SEQ ID NO: 1.

4. Polypeptide immunogène selon la revendication 1, où ledit polypeptide immunogène consiste en une séquence d'acides aminés avec jusqu'à 10 altérations des acides aminés de la SEQ ID NO: 1.

5. Polypeptide immunogène selon la revendication 1, où ledit polypeptide immunogène consiste en une séquence d'acides aminés avec jusqu'à 5 altérations des acides aminés de la SEQ ID NO: 1.

6. Polypeptide immunogène selon la revendication 5, où ledit polypeptide immunogène consiste en une séquence d'acides aminés avec jusqu'à 1 altération des acides aminés de la SEQ ID NO: 1.

7. Polypeptide immunogène selon la revendication 1, où ledit polypeptide immunogène consiste essentiellement en les acides aminés 3-260 de la SEQ ID NO: 1 ou 3-264 de la SEQ ID NO: 2.

8. Polypeptide immunogène selon la revendication 1, où ledit polypeptide immunogène consiste en une séquence d'acides aminés de la SEQ ID NO: 1.

9. Polypeptide immunogène selon l'une quelconque des revendications 1 à 8, où ledit polypeptide immunogène est substantiellement purifié.

10. Immunogène comprenant le polypeptide immunogène selon l'une quelconque des revendications 1 à 9, où ledit immunogène consiste en ledit polypeptide immunogène et en une ou plusieurs régions ou fractions supplémentaires jointes d'une manière covalente audit polypeptide immunogène à la terminaison carboxyle ou à la terminaison amino, où chaque région ou fraction est sélectionnée indépendamment parmi une région ou une fraction ayant au moins l'une des propriétés suivantes: améliore la réponse immune, facilite la purification ou facilite la stabilité du polypeptide.

11. Composition capable d'induire une réponse immune protectrice chez un patient, comprenant une quantité efficace sur le plan immunologique de l'immunogène selon l'une quelconque des revendications 1 à 10 et un véhicule pharmaceutiquement acceptable.

12. Composition selon la revendication 11, où ladite composition comprend en plus un adjuvant.

13. Acide nucléique comprenant un gène recombinant comprenant une séquence de nucléotides codant le polypeptide selon l'une quelconque des revendications 1 à 8.

14. Acide nucléique selon la revendication 13, où ledit acide nucléique est un vecteur d'expression.

15. Cellule recombinante comprenant un gène recombinant comprenant une séquence de nucléotides codant le polypeptide selon l'une quelconque des revendications 1 à 8.

16. Procédé de préparation d'un polypeptide de *S. aureus* qui fournit une immunité protectrice, comprenant les étapes consistant à:
(a) cultiver la cellule recombinante selon la revendication 15 dans des conditions dans lesquelles un polypeptide est exprimé et
(b) purifier ledit polypeptide.

17. Polypeptide immunogène selon l'une quelconque des revendications 1 à 8 à utiliser dans une méthode de traitement par thérapie.

18. Polypeptide immunogène à utiliser selon la revendication 17, où la thérapie est un traitement prophylactique d'infection par *S. aureus.*

19. Polypeptide immunogène à utiliser selon la revendication 18, où la thérapie est destinée à un être humain.

20. Utilisation d'un polypeptide immunogène selon l'une quelconque des revendications 1 à 8, pour la fabrication d'un médicament pour le traitement prophylactique d'une infection par *S. aureus.*

21. Utilisation selon la revendication 20, où le traitement prophylactique est destiné à un être humain.

22. Combinaison d'un polypeptide immunogène selon l'une quelconque des revendications 1 à 9 et d'un autre immunogène.


### SEQUENCE LISTING

<110> Merck & Co., Inc.
<120> POLYPEPTIDES FOR INDUCING A PROTECTIVE IMMUNE RESPONSE AGAINST STAPHYLOCOCCUS AUREUS
<130> 21490Y PCT
<150> 60/545,447
   <151> 2004-02-18
<160> 19
<170> FastSEQ for Windows Version 4.0
<210> 1
   <211> 260
   <212> PRT
   <213> Artificial Sequence
<220>
   <223> truncated derivative of sai-1
<400> 1
   Met Gly Thr Gln Val Ser Gln Ala Thr Ser Gln Pro Ile Asn Phe Gln
   1 5 10 15
   Val Gln Lys Asp Gly Ser Ser Glu Lys Ser His Met Asp Asp Tyr Met
   20 25 30
   Gln His Pro Gly Lys Val Ile Lys Gln Asn Asn Lys Tyr Tyr Phe Gln
   35 40 45
   Thr Val Leu Asn Asn Ala Ser Phe Trp Lys Glu Tyr Lys Phe Tyr Asn
   50 55 60
   Ala Asn Asn Gln Glu Leu Ala Thr Thr Val Val Asn Asp Asn Lys Lys
   65 70 75 80
   Ala Asp Thr Arg Thr Ile Asn Val Ala Val Glu Pro Gly Tyr Lys Ser
   85 90 95
   Leu Thr Thr Lys Val His Ile Val Val Pro Gln Ile Asn Tyr Asn His
   100 105 110
   Arg Tyr Thr Thr His Leu Glu Phe Glu Lys Ala Ile Pro Thr Leu Ala
   115 120 125
   Asp Ala Ala Lys Pro Asn Asn Val Lys Pro Val Gln Pro Lys Pro Ala
   130 135 140
   Gln Pro Lys Thr Pro Thr Glu Gln Thr Lys Pro Val Gln Pro Lys Val
   145 150 155 160
   Glu Lys Val Lys Pro Thr Val Thr Thr Thr Ser Lys Val Glu Asp Asn
   165 170 175
   His Ser Thr Lys Val Val Ser Thr Asp Thr Thr Lys Asp Gln Thr Lys
   180 185 190
   Thr Gln Thr Ala His Thr Val Lys Thr Ala Gln Thr Ala Gln Glu Gln
   195 200 205
   Asn Lys Val Gln Thr Pro Val Lys Asp Val Ala Thr Ala Lys Ser Glu
   210 215 220
   Ser Asn Asn Gln Ala Val Ser Asp Asn Lys Ser Gln Gln Thr Asn Lys
   225 230 235 240
   Val Thr Lys His Asn Glu Thr Pro Lys Gln Ala Ser Lys Ala Lys Glu
   245 250 255
   Leu Pro Lys Thr
   260
<210> 2
   <211> 264
   <212> PRT
   <213> S. aureus
<220>
<400> 2
   Met Gly Thr Gln Val Ser Gln Ala Thr Ser Gln Pro Ile Asn Phe Gln
    1               5                  10                  15
   Val Gln Lys Asp Gly Ser Ser Glu Lys Ser His Met Asp Asp Tyr Met
               20                  25                  30
   Gln His Pro Gly Lys Val Ile Lys Gln Asn Asn Lys Tyr Tyr Phe Gln
           35                  40                  45
   Ala Val Leu Asn Asn Ala Ser Phe Trp Lys Glu Tyr Lys Phe Tyr Asn
       50                  55                  60
   Ala Asn Asn Gln Glu Leu Ala Thr Thr Val Val Asn Asp Asp Lys Lys
   65                  70                  75                  80
   Ala Asp Thr Arg Thr Ile Asn Val Ala Val Glu Pro Gly Tyr Lys Ser
                   85                  90                  95
   Leu Thr Thr Lys Val His Ile Val Val Pro Gln Ile Asn Tyr Asn His
               100                 105                 110
   Arg Tyr Thr Thr His Leu Glu Phe Glu Lys Ala Ile Pro Thr Leu Ala
           115                 120                 125
   Asp Ala Ala Lys Pro Asn Asn Val Lys Pro Val Gln Pro Lys Pro Ala
       130                 135                 140
   Gln Pro Lys Thr Pro Thr Glu Gln Thr Lys Pro Val Gln Pro Lys Val
   145                 150                 155                 160
   Glu Lys Val Lys Pro Ala Val Thr Ala Pro Ser Lys Asn Glu Asn Arg
                   165                 170                 175
   Gln Thr Thr Lys Val Val Ser Ser Glu Ala Thr Lys Asp Gln Ser Gln
               180                 185                 190
   Thr Gln Ser Ala Arg Thr Val Lys Thr Thr Gln Thr Ala Gln Asp Gln
           195                 200                 205
   Asn Lys Val Gln Thr Pro Val Lys Asp Val Ala Thr Ala Lys Ser Glu
       210                 215                 220
   Ser Asn Asn Gln Ala Val Ser Asp Asn Lys Ser Gln Gln Thr Asn Lys
   225                 230                 235                 240
   Val Thr Lys Gln Asn Glu Val His Lys Gln Gly Pro Ser Lys Asp Ser
                   245                 250                 255
   Lys Ala Lys Glu Leu Pro Lys Thr
               260
<210> 3
   <211> 280
   <212> PRT
   <213> Artificial Sequence
<220>
   <223> amino His-tagged construct of SEQ ID NO: 1
<400> 3
   Met Gly Ser Ser His His His His His His Ser Ser Gly Leu Val Pro
    1               5                  10                  15
   Arg Gly Ser His Met Gly Thr Gln Val Ser Gln Ala Thr Ser Gln Pro
               20                  25                  30
   Ile Asn Phe Gln Val Gln Lys Asp Gly Ser Ser Glu Lys Ser His Met
           35                  40                  45
   Asp Asp Tyr Met Gln His Pro Gly Lys Val Ile Lys Gln Asn Asn Lys
       50                  55                  60
   Tyr Tyr Phe Gln Thr Val Leu Asn Asn Ala Ser Phe Trp Lys Glu Tyr
   65                  70                  75                  80
   Lys Phe Tyr Asn Ala Asn Asn Gln Glu Leu Ala Thr Thr Val Val Asn
                   85                  90                  95
   Asp Asn Lys Lys Ala Asp Thr Arg Thr Ile Asn Val Ala Val Glu Pro
               100                 105                 110
   Gly Tyr Lys Ser Leu Thr Thr Lys Val His Ile Val Val Pro Gln Ile
           115                 120                 125
   Asn Tyr Asn His Arg Tyr Thr Thr His Leu Glu Phe Glu Lys Ala Ile
       130                 135                 140
   Pro Thr Leu Ala Asp Ala Ala Lys Pro Asn Asn Val Lys Pro Val Gln
   145                 150                 155                 160
   Pro Lys Pro Ala Gln Pro Lys Thr Pro Thr Glu Gln Thr Lys Pro Val
                   165                 170                 175
   Gln Pro Lys Val Glu Lys Val Lys Pro Thr Val Thr Thr Thr Ser Lys
               180                 185                 190
   Val Glu Asp Asn His Ser Thr Lys Val Val Ser Thr Asp Thr Thr Lys
           195                 200                 205
   Asp Gln Thr Lys Thr Gln Thr Ala His Thr Val Lys Thr Ala Gln Thr
       210                 215                 220
   Ala Gln Glu Gln Asn Lys Val Gln Thr Pro Val Lys Asp Val Ala Thr
   225                 230                 235                 240
   Ala Lys Ser Glu Ser Asn Asn Gln Ala Val Ser Asp Asn Lys Ser Gln
                   245                 250                 255
   Gln Thr Asn Lys Val Thr Lys His Asn Glu Thr Pro Lys Gln Ala Ser
               260                 265                 270
   Lys Ala Lys Glu Leu Pro Lys Thr
           275                 280
<210> 4
   <211> 284
   <212> PRT
   <213> Artificial Sequence
<220>
   <223> amino His-tagged construct of SEQ ID NO: 2
<400> 4
   Met Gly Ser Ser His His His His His His Ser Ser Gly Leu Val Pro
    1               5                  10                  15
   Arg Gly Ser His Met Gly Thr Gln Val Ser Gln Ala Thr Ser Gln Pro
               20                  25                  30
   Ile Asn Phe Gln Val Gln Lys Asp Gly Ser Ser Glu Lys Ser His Met
           35                  40                  45
   Asp Asp Tyr Met Gln His Pro Gly Lys Val Ile Lys Gln Asn Asn Lys
       50                  55                  60
   Tyr Tyr Phe Gln Ala Val Leu Asn Asn Ala Ser Phe Trp Lys Glu Tyr
   65                  70                  75                  80
   Lys Phe Tyr Asn Ala Asn Asn Gln Glu Leu Ala Thr Thr Val Val Asn
                   85                  90                  95
   Asp Asp Lys Lys Ala Asp Thr Arg Thr Ile Asn Val Ala Val Glu Pro
               100                 105                 110
   Gly Tyr Lys Ser Leu Thr Thr Lys Val His Ile Val Val Pro Gln Ile
           115                 120                 125
   Asn Tyr Asn His Arg Tyr Thr Thr His Leu Glu Phe Glu Lys Ala Ile
       130                 135                 140
   Pro Thr Leu Ala Asp Ala Ala Lys Pro Asn Asn Val Lys Pro Val Gln
   145                 150                 155                 160
   Pro Lys Pro Ala Gln Pro Lys Thr Pro Thr Glu Gln Thr Lys Pro Val
                   165                 170                 175
   Gln Pro Lys Val Glu Lys Val Lys Pro Ala Val Thr Ala Pro Ser Lys
               180                 185                 190
   Asn Glu Asn Arg Gln Thr Thr Lys Val Val Ser Ser Glu Ala Thr Lys
           195                 200                 205
   Asp Gln Ser Gln Thr Gln Ser Ala Arg Thr Val Lys Thr Thr Gln Thr
       210                 215                 220
   Ala Gln Asp Gln Asn Lys Val Gln Thr Pro Val Lys Asp Val Ala Thr
   225                 230                 235                 240
   Ala Lys Ser Glu Ser Asn Asn Gln Ala Val Ser Asp Asn Lys Ser Gln
                   245                 250                 255
   Gln Thr Asn Lys Val Thr Lys Gln Asn Glu Val His Lys Gln Gly Pro
               260                 265                 270
   Ser Lys Asp Ser Lys Ala Lys Glu Leu Pro Lys Thr
           275                 280
<210> 5
   <211> 268
   <212> PRT
   <213> Artificial Sequence
<220>
   <223> carboxyl His-tagged construct of SEQ ID NO: 1
<400> 5
   Met Gly Thr Gln Val Ser Gln Ala Thr Ser Gln Pro Ile Asn Phe Gln
    1               5                  10                  15
   Val Gln Lys Asp Gly Ser Ser Glu Lys Ser His Met Asp Asp Tyr Met
               20                  25                  30
   Gln His Pro Gly Lys Val Ile Lys Gln Asn Asn Lys Tyr Tyr Phe Gln
           35                  40                  45
   Thr Val Leu Asn Asn Ala Ser Phe Trp Lys Glu Tyr Lys Phe Tyr Asn
       50                  55                  60
   Ala Asn Asn Gln Glu Leu Ala Thr Thr Val Val Asn Asp Asn Lys Lys
   65                  70                  75                  80
   Ala Asp Thr Arg Thr Ile Asn Val Ala Val Glu Pro Gly Tyr Lys Ser
                   85                  90                  95
   Leu Thr Thr Lys Val His Ile Val Val Pro Gln Ile Asn Tyr Asn His
               100                 105                 110
   Arg Tyr Thr Thr His Leu Glu Phe Glu Lys Ala Ile Pro Thr Leu Ala
   115 120 125
   Asp Ala Ala Lys Pro Asn Asn Val Lys Pro Val Gln Pro Lys Pro Ala
   130 135 140
   Gln Pro Lys Thr Pro Thr Glu Gln Thr Lys Pro Val Gln Pro Lys Val
   145 150 155 160
   Glu Lys Val Lys Pro Thr Val Thr Thr Thr Ser Lys Val Glu Asp Asn
   165 170 175
   His Ser Thr Lys Val Val Ser Thr Asp Thr Thr Lys Asp Gln Thr Lys
   180 185 190
   Thr Gln Thr Ala His Thr Val Lys Thr Ala Gln Thr Ala Gln Glu Gln
   195 200 205
   Asn Lys Val Gln Thr Pro Val Lys Asp Val Ala Thr Ala Lys Ser Glu
   210 215 220
   Ser Asn Asn Gln Ala Val Ser Asp Asn Lys Ser Gln Gln Thr Asn Lys
   225 230 235 240
   Val Thr Lys His Asn Glu Thr Pro Lys Gln Ala Ser Lys Ala Lys Glu
   245 250 255
   Leu Pro Lys Thr Leu Glu His His His His His His
   260 265
<210> 6
   <211> 395
   <212> PRT
   <213> Artificial Sequence
<220>
   <223> amino His-tagged construct of SEQ ID NO: 7
<400> 6
   Met His His His His His His Ser Ser Gly Leu Val Pro Arg Gly Ser
   1 5 10 15
   Gly Met Lys Glu Thr Ala Ala Ala Lys Phe Glu Arg Gln His Met Asp
   20 25 30
   Ser Pro Asp Leu Gly Thr Asp Asp Asp Asp Lys Ala Met Gly Thr Lys
   35 40 45
   His Tyr Leu Asn Ser Lys Tyr Gln Ser Glu Gln Arg Ser Ser Ala Met
   50 55 60
   Lys Lys Ile Thr Met Gly Thr Ala Ser Ile Ile Leu Gly Ser Leu Val
   65 70 75 80
   Tyr Ile Gly Ala Asp Ser Gln Gln Val Asn Ala Ala Thr Glu Ala Thr
   85 90 95
   Asn Ala Thr Asn Asn Gln Ser Thr Gln Val Ser Gln Ala Thr Ser Gln
   100 105 110
   Pro Ile Asn Phe Gln Val Gln Lys Asp Gly Ser Ser Glu Lys Ser His
   115 120 125
   Met Asp Asp Tyr Met Gln His Pro Gly Lys Val Ile Lys Gln Asn Asn
   130 135 140
   Lys Tyr Tyr Phe Gln Thr Val Leu Asn Asn Ala Ser Phe Trp Lys Glu
   145 150 155 160
   Tyr Lys Phe Tyr Asn Ala Asn Asn Gln Glu Leu Ala Thr Thr Val Val
   165 170 175
   Asn Asp Asn Lys Lys Ala Asp Thr Arg Thr Ile Asn Val Ala Val Glu
   180 185 190
   Pro Gly Tyr Lys Ser Leu Thr Thr Lys Val His Ile Val Val Pro Gln
           195                 200                 205
   Ile Asn Tyr Asn His Arg Tyr Thr Thr His Leu Glu Phe Glu Lys Ala
       210                 215                 220
   Ile Pro Thr Leu Ala Asp Ala Ala Lys Pro Asn Asn Val Lys Pro Val
   225                 230                 235                 240
   Gln Pro Lys Pro Ala Gln Pro Lys Thr Pro Thr Glu Gln Thr Lys Pro
                   245                 250                 255
   Val Gln Pro Lys Val Glu Lys Val Lys Pro Thr Val Thr Thr Thr Ser
                   260                 265                 270
   Lys Val Glu Asp Asn His Ser Thr Lys Val Val Ser Thr Asp Thr Thr
           275                 280                 285
   Lys Asp Gln Thr Lys Thr Gln Thr Ala His Thr Val Lys Thr Ala Gln
       290                 295                 300
   Thr Ala Gln Glu Gln Asn Lys Val Gln Thr Pro Val Lys Asp Val Ala
   305                 310                 315                 320
   Thr Ala Lys Ser Glu Ser Asn Asn Gln Ala Val Ser Asp Asn Lys Ser
                   325                 330                 335
   Gln Gln Thr Asn Lys Val Thr Lys His Asn Glu Thr Pro Lys Gln Ala
                   340                 345                 350
   Ser Lys Ala Lys Glu Leu Pro Lys Thr Gly Leu Thr Ser Val Asp Asn
           355                 360                 365
   Phe Ile Ser Thr Val Ala Phe Ala Thr Leu Ala Leu Leu Gly Ser Leu
       370                 375                 380
   Ser Leu Leu Leu Phe Lys Arg Lys Glu Ser Lys
   385                 390                 395
<210> 7
   <211> 350
   <212> PRT
   <213> S. aureus
<400> 7
   Met Thr Lys His Tyr Leu Asn Ser Lys Tyr Gln Ser Glu Gln Arg Ser
    1               5                  10                  15
   Ser Ala Met Lys Lys Ile Thr Met Gly Thr Ala Ser Ile Ile Leu Gly
               20                  25                  30
   Ser Leu Val Tyr Ile Gly Ala Asp Ser Gln Gln Val Asn Ala Ala Thr
           35                  40                  45
   Glu Ala Thr Asn Ala Thr Asn Asn Gln Ser Thr Gln Val Ser Gln Ala
       50                  55                  60
   Thr Ser Gln Pro Ile Asn Phe Gln Val Gln Lys Asp Gly Ser Ser Glu
   65                  70                  75                  80
   Lys Ser His Met Asp Asp Tyr Met Gln His Pro Gly Lys Val Ile Lys
                   85                  90                  95
   Gln Asn Asn Lys Tyr Tyr Phe Gln Thr Val Leu Asn Asn Ala Ser Phe
               100                 105                 110
   Trp Lys Glu Tyr Lys Phe Tyr Asn Ala Asn Asn Gln Glu Leu Ala Thr
           115                 120                 125
   Thr Val Val Asn Asp Asn Lys Lys Ala Asp Thr Arg Thr Ile Asn Val
       130                 135                 140
   Ala Val Glu Pro Gly Tyr Lys Ser Leu Thr Thr Lys Val His Ile Val
   145                 150                 155                 160
   Val Pro Gln Ile Asn Tyr Asn His Arg Tyr Thr Thr His Leu Glu Phe
                   165                 170                 175
   Glu Lys Ala Ile Pro Thr Leu Ala Asp Ala Ala Lys Pro Asn Asn Val
               180                 185                 190
   Lys Pro Val Gln Pro Lys Pro Ala Gln Pro Lys Thr Pro Thr Glu Gln
           195                 200                 205
   Thr Lys Pro Val Gln Pro Lys Val Glu Lys Val Lys Pro Thr Val Thr
       210                 215                 220
   Thr Thr Ser Lys Val Glu Asp Asn His Ser Thr Lys Val Val Ser Thr
   225                 230                 235                 240
   Asp Thr Thr Lys Asp Gln Thr Lys Thr Gln Thr Ala His Thr Val Lys
                   245                 250                 255
   Thr Ala Gln Thr Ala Gln Glu Gln Asn Lys Val Gln Thr Pro Val Lys
               260                 265                 270
   Asp Val Ala Thr Ala Lys Ser Glu Ser Asn Asn Gln Ala Val Ser Asp
           275                 280                 285
   Asn Lys Ser Gln Gln Thr Asn Lys Val Thr Lys His Asn Glu Thr Pro
       290                 295                 300
   Lys Gln Ala Ser Lys Ala Lys Glu Leu Pro Lys Thr Gly Leu Thr Ser
   305                 310                 315                 320
   Val Asp Asn Phe Ile Ser Thr Val Ala Phe Ala Thr Leu Ala Leu Leu
                   325                 330                 335
   Gly Ser Leu Ser Leu Leu Leu Phe Lys Arg Lys Glu Ser Lys
               340                 345                 350
<210> 8
   <211> 354
   <212> PRT
   <213> S. aureus
<400> 8
   Met Thr Lys His Tyr Leu Asn Ser Lys Tyr Gln Ser Glu Gln Arg Ser
   1               5                   10                  15
   Ser Ala Met Lys Lys Ile Thr Met Gly Thr Ala Ser Ile Ile Leu Gly
               20                  25                  30
   Ser Leu Val Tyr Ile Gly Ala Asp Ser Gln Gln Val Asn Ala Ala Thr
           35                  40                  45
   Glu Ala Thr Asn Ala Thr Asn Asn Gln Ser Thr Gln Val Ser Gln Ala
       50                  55                  60
   Thr Ser Gln Pro Ile Asn Phe Gln Val Gln Lys Asp Gly Ser Ser Glu
   65                  70                  75                  80
   Lys Ser His Met Asp Asp Tyr Met Gln His Pro Gly Lys Val Ile Lys
                   85                  90                  95
   Gln Asn Asn Lys Tyr Tyr Phe Gln Ala Val Leu Asn Asn Ala Ser Phe
               100                 105                 110
   Trp Lys Glu Tyr Lys Phe Tyr Asn Ala Asn Asn Gln Glu Leu Ala Thr
           115                 120                 125
   Thr Val Val Asn Asp Asp Lys Lys Ala Asp Thr Arg Thr Ile Asn Val
       130                 135                 140
   Ala Val Glu Pro Gly Tyr Lys Ser Leu Thr Thr Lys Val His Ile Val
   145                 150                 155                 160
   Val Pro Gln Ile Asn Tyr Asn His Arg Tyr Thr Thr His Leu Glu Phe
                   165                 170                 175
   Glu Lys Ala Ile Pro Thr Leu Ala Asp Ala Ala Lys Pro Asn Asn Val
               180                 185                 190
   Lys Pro Val Gln Pro Lys Pro Ala Gln Pro Lys Thr Pro Thr Glu Gln
           195                 200                 205
   Thr Lys Pro Val Gln Pro Lys Val Glu Lys Val Lys Pro Ala Val Thr
       210                 215                 220
   Ala Pro Ser Lys Asn Glu Asn Arg Gln Thr Thr Lys Val Val Ser Ser
   225                 230                 235                 240
   Glu Ala Thr Lys Asp Gln Ser Gln Thr Gln Ser Ala Arg Thr Val Lys
                   245                 250                 255
   Thr Thr Gln Thr Ala Gln Asp Gln Asn Lys Val Gln Thr Pro Val Lys
               260                 265                 270
   Asp Val Ala Thr Ala Lys Ser Glu Ser Asn Asn Gln Ala Val Ser Asp
           275                 280                 285
   Asn Lys Ser Gln Gln Thr Asn Lys Val Thr Lys Gln Asn Glu Val His
       290                 295                 300
   Lys Gln Gly Pro Ser Lys Asp Ser Lys Ala Lys Glu Leu Pro Lys Thr
   305                 310                 315                 320
   Gly Leu Thr Ser Val Asp Asn Phe Ile Ser Thr Val Ala Phe Ala Thr
                   325                 330                 335
   Leu Ala Leu Leu Gly Ser Leu Ser Leu Leu Leu Phe Lys Arg Lys Glu
               340                 345                 350
   Ser Lys
<210> 9
   <211> 358
   <212> PRT
   <213> Artificial Sequence
<220>
   <223> carboxyl His-tagged construct of SEQ ID NO: 7
<400> 9
   Met Thr Lys His Tyr Leu Asn Ser Lys Tyr Gln Ser Glu Gln Arg Ser
    1               5                  10                  15
   Ser Ala Met Lys Lys Ile Thr Met Gly Thr Ala Ser Ile Ile Leu Gly
               20                  25                  30
   Ser Leu Val Tyr Ile Gly Ala Asp Ser Gln Gln Val Asn Ala Ala Thr
           35                  40                  45
   Glu Ala Thr Asn Ala Thr Asn Asn Gln Ser Thr Gln Val Ser Gln Ala
       50                  55                  60
   Thr Ser Gln Pro Ile Asn Phe Gln Val Gln Lys Asp Gly Ser Ser Glu
   65                  70                  75                  80
   Lys Ser His Met Asp Asp Tyr Met Gln His Pro Gly Lys Val Ile Lys
                   85                  90                  95
   Gln Asn Asn Lys Tyr Tyr Phe Gln Thr Val Leu Asn Asn Ala Ser Phe
               100                 105                 110
   Trp Lys Glu Tyr Lys Phe Tyr Asn Ala Asn Asn Gln Glu Leu Ala Thr
           115                 120                 125
   Thr Val Val Asn Asp Asn Lys Lys Ala Asp Thr Arg Thr Ile Asn Val
       130                 135                 140
   Ala Val Glu Pro Gly Tyr Lys Ser Leu Thr Thr Lys Val His Ile Val
   145                 150                 155                 160
   Val Pro Gln Ile Asn Tyr Asn His Arg Tyr Thr Thr His Leu Glu Phe
                   165                 170                 175
   Glu Lys Ala Ile Pro Thr Leu Ala Asp Ala Ala Lys Pro Asn Asn Val
               180                 185                 190
   Lys Pro Val Gln Pro Lys Pro Ala Gln Pro Lys Thr Pro Thr Glu Gln
           195                 200                 205
   Thr Lys Pro Val Gln Pro Lys Val Glu Lys Val Lys Pro Thr Val Thr
       210                 215                 220
   Thr Thr Ser Lys Val Glu Asp Asn His Ser Thr Lys Val Val Ser Thr
   225                 230                 235                 240
   Asp Thr Thr Lys Asp Gln Thr Lys Thr Gln Thr Ala His Thr Val Lys
                   245                 250                 255
   Thr Ala Gln Thr Ala Gln Glu Gln Asn Lys Val Gln Thr Pro Val Lys
               260                 265                 270
   Asp Val Ala Thr Ala Lys Ser Glu Ser Asn Asn Gln Ala Val Ser Asp
           275                 280                 285
   Asn Lys Ser Gln Gln Thr Asn Lys Val Thr Lys His Asn Glu Thr Pro
       290                 295                 300
   Lys Gln Ala Ser Lys Ala Lys Glu Leu Pro Lys Thr Gly Leu Thr Ser
   305                 310                 315                 320
   Val Asp Asn Phe Ile Ser Thr Val Ala Phe Ala Thr Leu Ala Leu Leu
                   325                 330                 335
   Gly Ser Leu Ser Leu Leu Leu Phe Lys Arg Lys Glu Ser Lys Leu Glu
               340                 345                 350
   His His His His His His
           355
<210> 10
   <211> 843
   <212> DNA
   <213> Artificial Sequence
<220>
   <223> nucleic acid sequence encoding SEQ ID NO: 3
<400> 10
   atgggcagca gccatcatca tcatcatcac agcagcggcc tggtgccgcg cggcagccat 60
   atgggcacac aagtttctca agcaacatca caaccaatta atttccaagt gcaaaaagat 120
   ggctcttcag agaagtcaca catggatgac tatatgcaac accctggtaa agtaattaaa 180
   caaaataata aatattattt ccaaaccgtg ttaaacaatg catcattctg gaaagaatac 240
   aaattttaca atgcaaacaa tcaagaatta gcaacaactg ttgttaacga taataaaaaa 300
   gcggatacta gaacaatcaa tgttgcagtt gaacctggat ataagagctt aactactaaa 360
   gtacatattg tcgtgccaca aattaattac aatcatagat atactacgca tttggaattt 420
   gaaaaagcaa ttcctacatt agctgacgca gcaaaaccaa acaatgttaa accggttcaa 480
   ccaaaaccag ctcaacctaa aacacctact gagcaaacta aaccagttca acctaaagtt 540
   gaaaaagtta aacctactgt aactacaaca agcaaagttg aagacaatca ctctactaaa 600
   gttgtaagta ctgacacaac aaaagatcaa actaaaacac aaactgctca tacagttaaa 660
   acagcacaaa ctgctcaaga acaaaataaa gttcaaacac ctgttaaaga tgttgcaaca 720
   gcgaaatctg aaagcaacaa tcaagctgta agtgataata aatcacaaca aactaacaaa 780
   gttacaaaac ataacgaaac gcctaaacaa gcatctaaag ctaaagaatt accaaaaact 840
   tga                                                               843
<210> 11
   <211> 855
   <212> DNA
   <213> Artificial Sequence
<220>
   <223> nucleic acid sequence encoding SEQ ID NO: 4
<400> 11
   atgggcagca gccatcatca tcatcatcac agcagcggcc tggtgccgcg cggcagccat 60
   atgggcacac aagtttctca agcaacatca caaccaatta atttccaagt gcaaaaagat 120
   ggctcttcag agaagtcaca catggatgac tatatgcaac accctggtaa agtgattaaa 180
   caaaataata aatattattt ccaagctgta ttgaacaacg catcattctg gaaagaatac 240
   aaattttaca atgcaaacaa tcaagaatta gcaacaactg ttgttaacga tgataaaaaa 300
   gctgacacta gaacaatcaa tgttgctgtt gaacctgggt ataagagttt aactacaaaa 360
   gtacatattg tcgtgccaca aattaattat aatcatagat atactacgca tttagaattt 420
   gaaaaagcaa ttcctacatt agctgacgca gcaaaaccaa acaatgttaa accggttcaa 480
   ccaaaacctg ctcaacctaa aacacctact gagcaaacga aaccagttca acctaaagtt 540
   gaaaaagtta aacctgctgt aactgcacca agcaaaaatg aaaacagaca aactacaaaa 600
   gttgtaagta gtgaagctac aaaagatcaa agtcaaacac aaagtgctcg tacagtgaaa 660
   acaacacaaa cagctcaaga tcaaaataaa gttcaaacac ctgttaaaga tgttgcaaca 720
   gcgaaatctg aaagcaacaa tcaagctgta agtgacaata aatcacaaca aactaacaaa 780
   gttacaaaac aaaacgaagt tcataaacaa ggaccttcaa aagattctaa agctaaagaa 840
   ttaccaaaaa cttga                                                  855
<210> 12
   <211> 37
   <212> DNA
   <213> Artificial Sequence
<220>
   <223> primer
<400> 12
   gagatatacc atgggcacaa aacattattt aaacagt 37
<210> 13
   <211> 36
   <212> DNA
   <213> Artificial Sequence
<220>
   <223> primer
<400> 13
   ccggcggccc tcgagtttag attcttttct tttgaa 36
<210> 14
   <211> 37
   <212> DNA
   <213> Artificial Sequence
<220>
   <223> primer
<400> 14
   gagatatacc atgggcacaa aacattattt aaacagt 37
<210> 15
   <211> 39
   <212> DNA
   <213> Artificial Sequence
<220>
   <223> primer
<400> 15
   ccggcggccc tcgagttatt tagattcttt tcttttgaa 39
<210> 16
   <211> 41
   <212> DNA
   <213> Artificial Sequence
<220>
   <223> primer
<400> 16
   gagatatacc atgggcacac aagtttctca agcaacatca c 41
<210> 17
   <211> 36
   <212> DNA
   <213> Artificial Sequence
<220>
   <223> primer
<400> 17
   ggtggtgctc gagagttttt ggtaattctt tagctt 36
<210> 18
   <211> 41
   <212> DNA
   <213> Artificial Sequence
<220>
   <223> primer
<400> 18
   gagatatcat atgggcacac aagtttctca agcaacatca c 41
<210> 19
   <211> 39
   <212> DNA
   <213> Artificial Sequence
<220>
   <223> primer
<400> 19
   ggtggtgctc gagtcaagtt tttggtaatt ctttagctt 39